(19) Europäisches Patentamt
European Patent Office
Office européen des brevets

(11) EP 2 446 739 A1

(12) **EUROPEAN PATENT APPLICATION**
published in accordance with Art. 153(4) EPC

(43) Date of publication:
**02.05.2012 Bulletin 2012/18**

(21) Application number: **10792033.2**

(22) Date of filing: **21.06.2010**

(51) Int Cl.:
***A01K 67/027*** *(2006.01)*

(86) International application number:
**PCT/JP2010/060420**

(87) International publication number:
**WO 2010/150715 (29.12.2010 Gazette 2010/52)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **26.06.2009 JP 2009151726**

(71) Applicant: **The Nippon Synthetic Chemical Industry Co., Ltd.**
**Osaka-shi**
**Osaka 531-0076 (JP)**

(72) Inventors:
- **Shomura Yuzo**
  **Nishinomiya-shi**
  **Hyogo 662-0011 (JP)**
- **SHIBUTANI Mitsuo**
  **Kakogawa-shi**
  **Hyogo 675-0021 (JP)**
- **TANIGUCHI Taizo**
  **Kobe-shi**
  **Hyogo 653-0038 (JP)**
- **HARA Yasuo**
  **Ashigarakami-gun**
  **Kanagawa**
  **2590151 (JP)**

(74) Representative: **De Clercq, Ann G. Y.**
**De Clercq & Partners**
**Edgard Gevaertdreef 10a**
**9830 Sint-Martens-Latem (BE)**

# (54) NONHUMAN ANIMAL MODEL OF MYOCARDIAL INFARCTION AND METHOD FOR CONSTRUCTING SAME

(57) The present invention provides a non-human animal model of myocardial infarction, which can be created by a less invasive method, shows an extremely low mortality during and after surgical induction of myocardial infarction, ensures the blood reflow after necrosis of cardiac muscles caused by blocking blood flow, and therefore, is usable in topical therapy for myocardial infarction and studies on regenerative medicine, and a method for creating the non-human animal model. The creation method comprises: a step of administering a temporary embolic agent to a branch blood vessel of a heart coronary artery to block blood flow in the branch blood vessel; and a step of, after a definite period of time after said vascular branch blockage, administering a temporary embolic agent to the target coronary artery which is intended to induce myocardial infarction to block blood flow in the target coronary artery. Preferred is the branch blood vessel is a branch of the target coronary artery in which myocardial infarction is to be induced.

[Fig. 6]

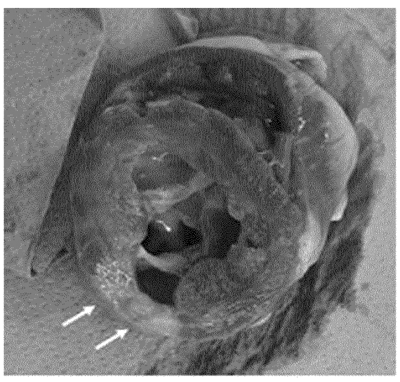



Printed by Jouve, 75001 PARIS (FR)

## Description

TECHNICAL FIELD

The present invention relates to a method of creating a non-human animal model of myocardial infarction with low mortality, and to a non-human animal model created by the method, which has an applicability to studies on topical therapy and regenerative medicine for myocardial diseases.

BACKGROUND ART

A non-human animal model which suffers from a pathological condition of myocardial infarction or myocardial ischemia is useful, such as for screening agents and establishing a method to treat pathological conditions including myocardial infarction or cardiac failure.

Animal models with such pathological conditions include, for example, a rat model in which a coronary artery is ligated permanently (coronary artery ligation model) as introduced in Japanese Unexamined Patent Publication No. 2002-291373 (patent document 1). However, such method of creating a model requires surgery with procedures including exposure of the heart and blood vessels through a thoracotomy under general anesthesia, as pointed out, for example, in Japanese Unexamined Patent Publication No. 2005-229927 (patent document 2), and such an invasive surgery imposes great burden on a study animal, and decreased pump function of the heart (heart failure) may gradually induce a shortage of blood flow in major organs such as lung and kidney other than the heart, and finally multiple organ failure may be occurred and so on, which leads to the death of 30 to 50% of the animals during or after the surgical treatment. In other words, the rate of successful creation of the model is very low.

Japanese Unexamined Patent Publication No. 2002-209473 (patent document 3) proposes a method for creating an animal model by carrying out a ligation surgery for the central part of the left coronary artery of the heart of a normal animal, conducting ectopic transplantation of the heart which was subjected to said coronary artery operation to the abdomen of another normal animal, and providing the resultant animal subjected to said ectopic heart transplantation for the pathological animal model of myocardial infarction. Such a method requires two animals for creating one model, therefore, it is not suitable for large-scale creation with large animals.

In the International Publication No. WO 2006-030737 (patent document 4), a method has proposed for creating a model which has a reduced mortality by ligating a blood vessel downstream of an artery, followed by embolizing the artery to be embolized.

There is a method recently used for interrupting the blood flow in a blood vessel with an ameroid ring (a plastic or metal ring with torus-shape casein core inside, in which the casein swells by absorbing body fluid and blocks the lumen of the blood vessel). By the method employing an ameroid ring, where the blood flow can be gradually decreased, as compared with the method in which ligation is performed using a suture or a clip, the method employing an ameroid ring enables the blood flow gradually decreased, and the mortality due to cardiac failure after the surgery can be decreased.

However, it is difficult to apply the method of interrupting blood flow using an ameroid ring to a downstream vessel ligation, therefore, also in the Example 1 of the above patent document 4, the ligation of a downstream blood vessel is carried out with a suture, followed by blocking a coronary artery by using an ameroid ring.

[Patent document 11 Japanese Unexamined Patent Publication No.2002-291373A
[Patent document 2] Japanese Unexamined Patent Publication No.2005-229927A
[Patent document 3] Japanese Unexamined Patent Publication No.2002-209473A
[Patent document 4] Japanese International Publication No.WO2006-030737

DISCLOSURE OF THE INVENTION

PROBLEMS TO BE SOLVED BY THE INVENTION

In the method proposed by the patent document 4, it is intended that local ligation in advance provides the tolerance to ischemia in the whole cardiac muscle to increase the rate of survival of animals which has undergone surgery inducing myocardial infarction, however, the interruption of blood flow in 2 times in the above method both involve thoracotomy, thus invasive surgeries impose a big burden on animals, and the mortality risk of animals during creating the model is high.

Moreover, in the myocardial infarction model where the blood flow is interrupted by ligation, a catheter cannot be inserted through the lumen of the blood vessel ligated, therefore, the model cannot be utilized as a study model for

local myocardial regenerative medicine.

Interruption of blood flow by an ameroid ring employs swelling of casein in the interior side of the ring by absorbing fluid, and thus it is difficult to adjust the rate of expansion, and for this reason, there is a wide variation in the time required for blocking blood flow and it is not easy to create a desired myocardial infarction model stably in terms of time.

According to the above patent document 4, a method of blocking an artery by forming an autologous blood clot is proposed as a method of interrupting blood flow without using ligation.

As an example 3, a cerebral arterial embolus model was generated by adding a coagulating agent (ADP or thrombin) to autologous blood to form an autologous blood clot (thrombus), and subsequently delivering the autologous blood clot to the lumen of the artery of interest using a catheter to interrupt the blood flow.

In the method of interrupting blood flow by using an autologous blood clot, to rapidly block a blood vessel with large caliber, such as a coronary artery, a large amount of blood clots have to be injected, and thus, it is not easy to block a normal coronary artery entirely only by using blood clots, and creating a stable model is difficult. Furthermore, a part of the blood clot possibly becomes organized by biological reactions to block a blood vessel permanently, and because of the difference in thrombus resolution time between individuals or sites, it is difficult to create a myocardial infarction model efficiently with good reproducibility for different sites.

The present invention has been made under these circumstances, and the object of the invention is to provide a non-human animal model of myocardial infarction, which can be created by a less invasive method, shows an extremely low mortality during and after surgical induction of myocardial infarction, ensures the blood reflow after necrotizing cardiac muscles by interrupting the blood supply, and therefore, is usable in topical therapy for myocardial infarction and studies on regenerative medicine, and a method for creating same.

## MEANS FOR SOLVING THE PROBLEMS

The present invention provides a method for creating a non-human animal model of myocardial infarction comprising a first and a second vascular blockages. The first blockage, referred to as vascular branch blockage, is performed by administering a temporary embolic agent to a branch blood vessel of a heart coronary artery to block the branch blood vessel. The second blockage is performed by, after a definite period of time after said vascular branch blockage, administering a temporary embolic agent to a target coronary artery which is intended to induce myocardial infarction to block the target coronary artery. Preferred is that said branch blood vessel is a branch blood vessel of the target coronary artery which is intended to induce myocardial infarction.

In a preferable embodiment, at least one selected from the group consisting of said first and said second vascular blockages is carried out by administering a temporary embolic agent into a blood vessel with expanding a balloon catheter at a upstream of the blood vessel.

The temporary embolic agent is preferably in a form of dispersion or solution or paste in which spherical PVA particles are dispersed or dissolved in a medium.

The present invention also provides a myocardial infarction non-human animal model created by the inventive method. The non-human animal model of the invention is characterized by comprising a pathological site of myocardial infarction wherein at least one blood vessel of a coronary artery is suffered from ischemia to cause necrosis of a myocardial cell but the blood vessel is able to be revascularized. The preferable non-human animal is a pig.

The present invention further provides a method of blocking blood flow of a non-human animal. The method is characterized by administering spherical PVA micro particles in an amount sufficient to embolize a blood vessel to be intended to block blood flow therein while the blood flow is inhibited by inserting a balloon catheter to an entrance of the blood vessel and expanding the balloon catheter at the entrance.

## EFFECT OF THE INVENTION

According to the inventive method of creating a myocardial infarction non-human animal model, the model can be created by a less invasive surgery without thoracotomy, and the mortality risk of the animal during the surgery is low. In addition, since the inventive method does not use an embolic agent such as thrombus that has differences among individuals, the method can provide a stable and good reproducible model.

Furthermore, the inventive myocardial infarction non-human animal model can revascularize the blood vessel where ischemia has been induced at a focus site of myocardial infarction, and therefore, the model can be utilized in screening therapeutic drugs for myocardial infarction, as well as in studies on other field concerning myocardial infarction including regenerative medicine.

BRIEF DESCRIPTION OF DRAWING

Figure 1 is a coronary angiograph of the heart of model No.1 in Example in a state of carrying out the step for blocking a branch blood vessel, which was taken in an early stage of administering a temporary embolic agent.
Figure 2 is a coronary angiograph of the heart of model No. 1 in Example in a state of carrying out the step for blocking a branch blood vessel, which was taken immediately before the completion of administration.
Figure 3 is a coronary angiograph of the heart of model No.1 in Example in a state of carrying out the step for inducing myocardial infarction, which was taken in an early stage of administering a temporary embolic agent.
Figure 4 is a coronary angiograph of the heart of model No.1 in Example in a state of carrying out the step for inducing myocardial infarction, which was taken immediately before the completion of administration.
Figure 5 shows a visible image obtained by contrast MRI scans of model No.1 in Example. The contrast MRI scans were conducted at 1 week after inducing myocardial infarction.
Figure 6 is a photograph of the heart of the model No.1 in Example, which was taken using a camera with macroscopic lens at 1 week after inducing myocardial infarction.
Figure 7 shows a long-axis image obtained by contrast MRI scans of the heart of the model No.6 in Example, which was taken at 1 week after inducing myocardial infarction.
Figure 8 shows a short-axis image obtained by contrast MRI scans of the heart of the model No.6 in Example, which was taken at 1 week after inducing myocardial infarction.
Figure 9 is a coronary angiograph of descending branch of coronary artery of the model No.8 in Example, which was taken after embolizing the descending of coronary artery.

BEST MODE FOR CARRYING OUT OF THE INVENTION

Hereinafter, the present invention will be described in detail. The description below merely illustrates one embodiment (typical embodiment) of the present invention, and this should not be construed as limiting the scope of the invention.

<Creation of a model of myocardial infarction>

The method for creating a non-human animal model of myocardial infarction of the present invention comprises a first and a second vascular blockages. The first vascular blockage, which corresponds to a step for preconditioning, is performed by administering a temporary embolic agent to a branch blood vessel of a heart coronary artery to block blood flow through the branch blood vessel. The second vascular blockage, which corresponds to a step for inducing myocardial infarction, is performed by administering a temporary embolic agent to a target coronary artery intended to cause myocardial infarction after a definite period of time after said preconditioning, to block blood flow through the target coronary artery.

A target coronary artery of the present invention is a coronary artery in which myocardial infarction can be induced, including left anterior descending branch (LAD), left circumflex branch (LCX), left main trunk (LMT) and right coronary artery (RCA).
Rapid decrease or interruption of the blood flow in at least one of these blood vessels causes myocardial ischemia to necrosis, which is so called myocardial infarction.

[Step for vascular branch blockage (preconditioning)]

A blood vessel blocked in the step for blockage branch vascular is any one of branch blood vessels of the coronary artery described above. The branch blood vessel may not be a branch blood vessel of the coronary artery which is intended to cause myocardial infarction.

In spite of the interruption of blood flow in a branch blood vessel, if its transflux region is small, a serious condition of myocardial infarction is not often brought on, and then the tolerance to ischemia can be imparted to the entire cardiac muscle. Even though the branch blood vessel to be blocked in the preconditioning step is not a branch of the target coronary artery in which myocardial infarction is to be induced, it is presumed that the tolerance to ischemia to the entire cardiac muscle can be imparted. However, it is desired that the branch blood vessel blocked in said step is a branch of the target coronary artery causing myocardial infarction, in order to minimize the influence on the cardiac muscle in the sites other than the site of myocardial infarction to be induced.

In administering a temporary embolic agent to a branch blood vessel, reflux of blood or the like possibly let the temporary embolic agent flow into some branch blood vessels other than the target branch, which leads to embolization

of the blood vessels other than the target blood vessel, or shortage of the temporary embolic agent to embolize the target branch, therefore, it is preferable to temporarily block the entrance of the target branch blood vessel in advance with a first balloon catheter, and subsequently administer a temporary embolic agent inside the blood vessel at a distal site in the downstream blood vessel blocked by the balloon.

A balloon catheter is typically inserted through a femoral artery of four limbs toward the heart. By applying this way, a use of a highly invasive surgical procedure of thoracotomy can be avoided.

The first balloon catheter may be designed so that the balloon at the tip of the catheter reaches a certain diameter when a certain pressure given. A type of the first balloon catheter is not specifically limited, but an over-the-wire catheter, in which the balloon of the catheter is positioned along a guide wire, is preferably used. The diameter of the balloon may be selected according to the diameter of the blood vessel to be blocked.

The time to interrupt blood flow by a balloon catheter only have to be longer than the time required to block the blood vessel after the administration of a temporary embolic agent, it is typically 15 to 60 minutes. The major purpose of the balloon expansion in this step is to prevent backflow of the embolic agent administered, and not to induce tissue necrosis by interrupting the blood flow, therefore, the duration of the time noted above is sufficient.

In a preferred embodiment, a temporary embolic agent is administered with a catheter. The catheter used herein is preferably the same as the one used for the above first balloon catheter. By this usage, the manipulation of catheter insertion can be conducted by one guide wire, and thus a balloon expansion may be followed by the release of an agent from the channel for the guide wire, which leads to the increase of efficiency of operation.

It is preferable that a temporary embolic agent is administered while washing the catheter with saline. Specifically, after a temporary embolic agent is administered, saline is injected, or alternatively, a required amount of a temporary embolic agent is divided into multiple portions, and administration of a temporary embolic agent and injection of saline are carried out alternately. By this operation, the embolic agent remained or attached in the catheter can be washed out and a given amount of a temporary embolic agent may be precisely administered and also the remained blood inside the blood vessel may be washed out.

In advance of administering a temporary embolic agent, an anticoagulant such as heparin and the like is preferably administered. It is to prevent formation of a blood clot, which resulted from contacting of blood with an exogenous substance such as a temporary embolic agent.

It is preferred that the operations described above are performed while being checked by angiography, and administering may be continued until angiography confirms that the blood flow is stopped. A contrast agent may be administered separately from a temporary embolic agent, or contained in a temporary embolic agent.

[Step of inducing myocardial infarction]

After a definite period of time after aforementioned vascular branch blockage, a step for blocking blood flow in a target coronary artery is conducted.

Herein, the definite period of time is typically from 1 to 10 days. This is almost the period in which an embolic agent remains in a branch blood vessel and also is able to block blood flow in the vicinity of the branch blood vessel, and so, the tolerance of cardiac muscle to ischemia is developed to some extent.

During the period, the temporary embolic agent used for blocking the branch blood vessel is degraded or dissolved in body fluids, and excreted out of the body.

The step for blocking blood flow in a target coronary artery is performed by administering a temporary embolic agent to the target coronary artery, more specifically, an artery from which a branch blood vessel is derived, including left anterior descending branch (LAD), left circumflex branch (LCX), left main trunk (LMT) or right coronary artery (RCA). In a preferable embodiment, the step for blocking blood flow of a target coronary artery is performed in a similar manner as the step for blocking a branch blood vessel in order to inhibit the reflux of a temporary embolic agent or the like. That is, the step is preferably to temporarily block the entrance of the coronary artery with a second balloon catheter, and subsequently administer a temporary embolic agent at a distal site in the downstream blood vessel blocked by the balloon.

In a manner similar to the blood flow interruption by the 1st balloon catheter, the blood flow interruption by the 2nd balloon catheter can be normally conducted by inserting a guiding catheter beforehand to the proximity of the root of a targeted artery, leaving the guiding catheter in place, inserting the 2nd balloon catheter through the guiding catheter, projecting the balloon from the tip and expanding the balloon.

A temporary embolic agent is administered by a catheter which is preferably the same as the one used for the 2nd balloon catheter, also in a similar manner as the step for blocking a branch blood vessel. And it is preferred that the administering is conducted while washing the catheter with saline.

The 2nd balloon catheter can be the one with a balloon diameter to interrupt the blood flow of a coronary artery. A temporary embolic agent cannot be injected for blocking in the part of the balloon expanded, therefore, the length of balloon is preferably short. Typically, a balloon having a length of 1 to 2 cm is used.

A temporary embolic agent to be administered is preferably the same kind of the temporary embolic agent

used in the step for blocking a branch blood vessel. In advance of administering a temporary embolic agent, an anticoagulant is preferably administered. In addition, preferred is that a temporary embolic agent is administered while washing the catheter with saline.

In a similar manner as the step for blocking a branch blood vessel, it is preferred that the operations described above are performed while being checked by angiography, and administering may be continued until angiography confirms the stop of the blood flow. A contrast agent may be administered in advance of administration of a temporary embolic agent, or together with a temporary embolic agent.

In the step to induce myocardial infarction as described above, cardiac arrhythmia, fall in blood pressure or the like are rarely observed, and the mortality rate of non-human animal is extremely low.

After certain period of time (about one week) after the step of induction of myocardial infarction described above, a disease model of myocardial infarction is created. Confirmation of myocardial infarction can be performed by contrast MRI scans. When the site with delayed excretion of a contrast agent is confirmed, it is thought that a focus of myocardial infarction was formed according to the image diagnosis. The survival rate of model animals until formation of a focus of myocardial infarction is not less than 90%. It is considered that the ability of cardiac muscle for responding to ischemia was increased through a step for blocking a branch blood vessel and being left for a lapse of specific time (preconditioning), therefore, the mortality due to myocardial infarction induced over a larger area was reduced.

< temporary embolic agent >

A temporary embolic agent used in the invention is in a form of dispersion liquid, solution or paste in which a temporary embolic material is dispersed or dissolved in a medium. The temporary embolic material can embolize a target blood vessel to inhibit or block blood flow, and can dissolve in the body fluid over certain period of time, thereby avoiding permanent blockage of blood flow because of not remaining in the body.

An embolization time of a temporary embolic agent is a time of period required for attaining the pathological condition of myocardial infarction. The embolization time is not limited to, but is usually between 1 and 14 days in the case that an embolic agent is used for creating a myocardial infarction model of the invention. A temporary embolic agent may be block and/or inhibit blood flow during the embolization period.

A temporary embolic material may be, for instance, a gelatin sponge disclosed in International Publication No.WO98/3203; an almost spherical particle having water-swelling rate of 30% or more and degradability in phosphate buffered saline at 37˚C disclosed in Japanese Unexamined Patent Publication No. 2004-167229; pearl shape PVA particle having a saponification degree of 90 mol% or more and an average particle diameter of 70 to 1000 μm disclosed in Japanese Unexamined Patent Publication No.2007-37989.
A spherical PVA particle is preferred in the view that the spherical PVA particle can be dissolved in blood over certain period of time to revascularize the embolized blood vessel, resulting in blood reflow and preventing from remaining the foreign material in the body.
A spherical PVA particle, which is a particularly preferable temporary embolic material, will be explained below.

[spherical PVA particle]

PVA as used herein includes a saponified polyvinyl ester polymer (unmodified PVA), which is a saponified product of homo- or copolymer of vinyl ester such as vinyl acetate, vinyl propionate, vinyl formate, vinyl stearate, vinyl benzonate; a saponified vinyl ester-based polymer (pre-modified PVA), which is a saponified product of copolymer obtained by copolymerizing vinyl ester and a monomer copolymerizable therewith; and a modified product (post-modified PVA) obtained by modifying a saponified unmodified PVA with sulfonic acid, carboxylic acid and so on.

The copolymerizable monomer with vinyl ester includes, for instance, unsaturated acid such as acrylic acid, methacrylic acid, crotonic acid, maleic acid, maleic anhydride, and itaconic acid, and a salt thereof, and mono- or dialkyl ester of unsaturated carboxylic acid; α-olefins such as ethylene, propylene, and so on; hydroxy group-containing α-olefins such as 3-buten-1-ol, 4-penten-1-ol, 5-hexen-1-ol, 3,4-dihydroxy-1-butene, and its derivatives such as acyl thereof alicyclic hydrocarbon such as norbornene; nitrile such as acrylonitrile, methacrylonitrile; amide such as diacetone acrylamide, acrylamide, methacrylamide; olefinsulfonic acid such as ethylenesulfonic acid, allylsulfonic acid, methallyl sulfonic acid and a salt thereof; alkylvinyl ether; dimethyl allyl vinyl ketone, N-vinyl pyrrolidone, (meth)acrylate; as well as cation group-containing monomer such as N-acrylamide methyl trimethyl ammoniumchloride, N-acrylamide ethyl trimethyl ammoniumchloride, N-acrylamide propyl trimethyl ammoniumchloride, 2-acryloxy ethyl trimethyl ammoniumchloride, 2-methacryloxy ethyl trimethyl ammoniumchloride, 2-hydroxy-3-methacryloyloxy propyl trimethyl ammoniumchloride, allyl trimethyl ammoniumchloride, methallyl trimethyl ammoniumchloride, 3-butene trimethyl ammoniumchloride, dimethyl diallyl ammonium chloride, diethyl diallyl ammoniumchloride; acetoacetyl group-containing monomer; allylsulfonic acid, 2-acrylamide-2-methyl propene sulfonic acid, glycerin monoallylether, isopropenyl acetate, 1-methoxy vinyl acetate, 1,4-diacetoxy-2-butene, and so on.

The modified PVA includes a saponified copolymer containing the copolymerizable monomer unit, as well as a PVA containing the 1,2-diol unit in a side chain shown in the formula 1 below.

[formula 1]

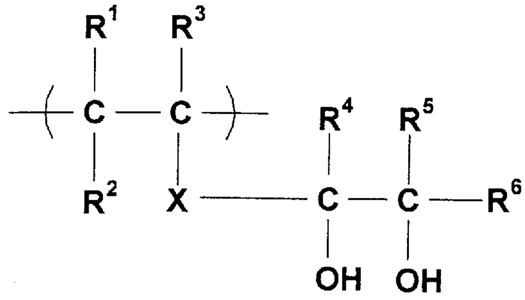

(1)

In the formula 1, each of $R^1$ to $R^6$ represents individually hydrogen atom or organic group. It is preferred that all of $R^1$ to $R^6$ are hydrogen atoms, however, each of $R^1$to $R^6$can be an organic group unless it causes to drastically impair properties as the PVA resin. Preferably, the organic group includes, for instance, but is not limited to, alkyl group having 1 to 4 carbon atoms such as methyl, ethyl, n-propyl, isopropyl, n-butyl, isobutyl, tert-butyl. The alkyl group may have a substituent group such as halogen, hydroxyl group, ester group, carboxylic acid group, and sulfonic acid group, according to needs.

In the formula 1, X is single bond or binding group, preferably single bond from the viewpoint of solubility and degree of crystallization. The binding group includes, but is not limited to, hydrocarbon such as alkylene, alkenylene, alkynylene, phenylene, naphthylene and so on (which may be substituted with halogen such as fluorine, chlorine, bromine etc.), as well as -O-, $-(CH_2O)m-$, $-(OCH_2)m-$, $-(CH_2O)mCH_2-$, -CO-, -COCO-, $-CO(CH_2)mCO-$, $-CO(C_6H_4)CO-$ -S-, -CS-, -SO-, $-SO_2-$, -NR-, -CONR-, -NRCO-, -CSNR-, -NRCS-, -NRNR-, $-HPO_4-$, $-Si(OR)_2-$, $-OSi(OR)_2-$, $-OSi(OR)_2O-$, -Ti $(OR)_2-$, $-OTi(OR)_2-$, $-OTi(OR)_2O-$, -Al(OR)-, OAl(OR)-, -OAl(OR)O-, wherein R is individually any substituent group, preferably hydrogen atom, or alkyl group, and m is positive integer. Of these, preferred is alkylene group having 6 or less carbon atoms, particularly, methylene group or $-CH_2OCH_2-$ in view of heat resistance and stability of viscosity.

As the modified PVA, a modification degree is in the range of usually 0.2 to 1 mol%, preferably 0.1 to 1 mol%, and more preferably 0.3 to 0.5 mol%.

A degree of saponification of vinyl ester in the PVA used herein is usually 90 mol% or more, as a value measured according to JIS K6726. Specifically, the degree of saponification is selected from the range of 90 to 99.5 mol% in the case of an unmodified PVA, and 99 mol% or more in the case of a modified PVA for the following reason.

Solubility of unmodified PVA is controlled by its saponification degree, while solubility of modified PVA is mainly controlled by its modification amount and a condition of heat treatment. Accordingly, unmodified PVA having relatively wide range of saponification degree is preferably used, while modified PVA with a high saponification degree is preferably used because the saponification degree has slight influence on solubility.

A degree of crystallization is controlled preferably by a modification amount because the crystalline can be controlled by modification more delicately than by a saponification degree, and the crystalline controlled by modification is more excellent in reproducibility, although depending on a type of modification.

PVA having an insufficient saponification degree is easily swollen by an aqueous medium to increase the viscosity of the agent enough to bring about deteriorating its passing property in catheter as follows. The swollen PVA particles would embolize in the catheter, and a pressure necessary for administering embolic agent becomes so high that manipulability is drastically lowered, which is unfavorable.

In a particularly preferable embodiment, spherical PVA particles used as a temporary embolic agent is a spherical particle made of the above-mentioned PVA, and also having the following characters.

A diameter of the spherical PVA particle is preferably in the range of 50 to 800 $\mu$m, more preferably 100 to 250 $\mu$m, and further more preferably 105 to 212 $\mu$m.

The diameter of the coronary artery is usually from 500 to 3000 $\mu$m, while a diameter of the branch of coronary artery is in the range of 50 to 1000 $\mu$m. Accordingly, when a spherical PVA particle has too small average particle diameter, capillaries as well as a blood vessel other than the target blood vessel is easily embolized, which is unfavorable. On the other hand, when a spherical PVA particle has too large average particle diameter, it would cause to remarkably lower its passing property in some catheters or would not be able to pass through in some catheters, depending on the types of the catheters used.

An average polymerization degree of the spherical PVA particle used herein is usually in the range of 80 to

1500, preferably 90 to 1000, more preferably 100 to 800, and further more preferably 300 to 600. The spherical PVA particle having unduly low average polymerization degree is not easy to be produced industrially and stably. In addition, the spherical PVA particle having unduly low average polymerization degree could dissolve in body fluid rapidly to be exhausted before inducing myocardial infarction. While a spherical PVA particle having unduly high average polymerization degree dissolves very slowly in blood vessel, resulting in deteriorating a function of the temporary embolic agent using the spherical PVA particle.

Preferably, the spherical PVA particle used herein has preferably a shape close to true sphere. A spherical PVA particle having an almost spherical shape shows excellent catheter passing property and lessens injury in blood vessel and can achieve complete vascular blockage more effectively.
Specifically, the spherical degree is usually 0.96 or more, preferably 0.98 or more, and furthermore preferably 0.99 or more. A polyvinyl alcohol particle having such an almost sphere shape shows excellent passing property in catheter and secure a smooth manipulability in administration of them.

A degree of crystallization of the spherical PVA particle used herein is preferably in the range of 55 to 65%, more preferably 58 to 63%, and further more preferably 60 to 62 %. The degree of crystallization of spherical PVA particle may be controlled by a condition of heat treatment after preparation of PVA. When the degree of crystallization is unduly low, spherical PVA particle would be easily swollen by water in catheter, resulting in lowered catheter passing property, while the degree of crystallization is unduly high, the dissolution rate of the spherical PVA particle would become too slow. In order to adjust the degree of crystallization, the heat treatment is usually conducted at a temperature between 120 and 150°C, preferably between 130 and 140°C.

Preferably, spherical PVA particles used herein is adjusted such that solution speeds in water and in blood are individually within the ranges as follows.
The dissolution speed in water is selected in the range of usually 25 to 70%, preferably 30 to 60%, and more preferably 30 to 55%, in terms of residual rate as measured in the following method. The residual rate for evaluation index of solution speed in water is measured by charging 3 g of PVA particles classified into 100 to 212 μm and 70g of water into 100 ml of beaker, stirring for two minutes at room temperature, and leaving the beaker in water bath set at a temperature of 37°C, followed by counting the number of spherical PVA particles with stirring using LASENTEC M100F (manufactured by Lasentec, take-in time: 24.75 seconds). The residual rate is calculated as a ratio of spherical PVA particle numbers of 3 hours after starting measurement relative to immediately after starting measurement.

The spherical PVA particle having the aforementioned properties may be produced by, for instance, a method for producing a spherical polyvinyl alcohol particle disclosed in Japanese Unexamined Patent Publication No.S56-120707. The method is as follows: polyvinyl ester solution (b) (solvent: alcohol or a mixture of alcohol and methyl acetate (a)), is granularly dispersed in a medium (c), which is substantially incompatible with any of polyvinyl ester, saponified product thereof, and the component (a), and which is more viscous than the component (b). The dispersion is subjected to saponification in the presence of saponification catalyst to obtain PVA particles.

An alcohol as the component (a) may be a lower aliphatic alcohol such as methanol, ethanol, isopropyl alcohol, propyl alcohol and a mixture of two or more thereof in an arbitrary mixing ratio. Among them, methanol, ethanol and isopropyl alcohol are preferably used in the point of controllability of particle diameter during saponification and practically employable speed of saponification. In the case of using alcohol with methyl acetate together, the mixing weight ratio of alcohol/methyl acetate is preferably 0.5 or more, and more preferably 1.5 or more, in the point of efficiency of saponification of polyvinyl ester. It is possible to use a variety of organic solvents having lower polarity than methyl acetate together.

The polyvinyl ester content in the component (b) is not limited to, but usually within the range of 10 to 80 % by weight based on total solvent. The component (b) (polyvinyl ester solution) may contain water in an amount of 0.05 to 10 parts by weight relative to polyvinyl ester. A small quantity of water may distribute residual acetic acid groups randomly in the saponified product, and make a role to adjust a saponification degree of the component (b).

The medium (c) is substantially incompatible with any of polyvinyl ester, saponified product thereof, and component (a) (alcohol or a mixture of alcohol and methyl acetate) and more viscous than polyvinyl ester solution (b). Examples of the medium (c) include aliphatic saturated hydrocarbon such as liquid paraffin and kerosine; aromatic hydrocarbon; and alicyclic hydrocarbon. These may be usable alone or in a mixture of two or more of them. Particularly, liquid paraffin may be preferably used in view of homogenously dispersing polyvinyl ester solution therein.

The viscosity of medium (c) is not specifically limited as far as it is higher than the viscosity of polyvinyl ester solution (b).

The ratio of polyvinyl ester solution (b) to medium (c) is selected form the range of 2/8 to 6/4, preferably 4/6 to 5/5, in terms of weight ratio. Less than 20 % by weight of polyvinyl ester solution (b) is unfavorable because of reduction of production efficiency. When the proportion of polyvinyl ester solution (b) exceeds 60 % by weight, dispersibility becomes poor, as a result, agglomerate of a lot of particles is easily formed and an average particle diameter of spherical PVA particle tends to be enlarged.

An ordinary alkaline catalyst used for saponifying polyvinyl ester to produce PVA may be used for the saponification catalyst. An amount of the saponification catalyst is properly selected depending on the concentration of polyvinyl ester and the desired degree of saponification, is usually in the range of 0.1 to 30 mmol, and preferably 2 to 17 mmol, relative to vinyl acetate unit (1 mol) of polyvinyl ester.

Saponification is preferably conducted at a temperature selected from the range of 20°C to 60°C. The reaction temperature of not more than 20°C causes to slow down the reaction speed, additionally the reaction efficiency is lowered. The reaction temperature exceeding 60°C is unfavorable in view of safety, because the reaction temperature is higher than the boiling point of the solvent.

Spherical PVA particle having a high saponification degree of 99.0% or more is preferably produced by two-stage saponification from the viewpoint of properties of spherical PVA particles to be obtained and safety by reduction of toxicity to human body due to liquid paraffin taken in spherical PVA particles during production. The primary saponification is carried out until the saponification degree is reached between 75 to 90 mol%, and the resultant particles are separated from the reaction slurry by filtration with a solid-liquid separator such as centrifugal separator, or filtration with Advantec filter paper No.2 or No.63 in the case of laboratory work, followed by rinsing with an appropriate solvent such as methanol, methyl acetate, ethyl acetate, or a mixed solvent thereof such as a mixture of methyl acetate and methanol, according to needs. Subsequently, the obtained primary saponified particle is dispersed in a solvent containing alcohol such as methanol, ethanol etc., followed by further proceeding saponification reaction. When the desired degree of saponification is attained, the reaction is terminated, and spherical PVA particle (secondary saponified particle) is obtained in the same manner as to the collection of the primary saponified particles. After then, the obtained spherical PVA particles are rinsed with saline, ethanol and so on, if necessary.

A method for producing PVA containing 1,2-diol in a side chain preferably includes, but is not limited to, (i) a method of saponifying a copolymer of vinyl ester-based monomer and a compound represented by the following formula 2; (ii) a method of saponifying and decarboxylating a copolymer of vinyl ester-based monomer and a compound represented by the following formula 3; (iii) a method of saponifying and deketalizing a copolymer of vinyl ester-based monomer and a compound represented by the following formula 4.

[formula 2]

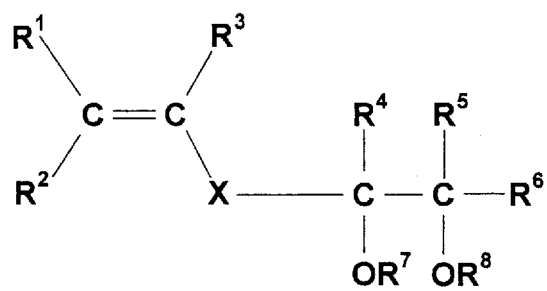

(2)

[formula 3]

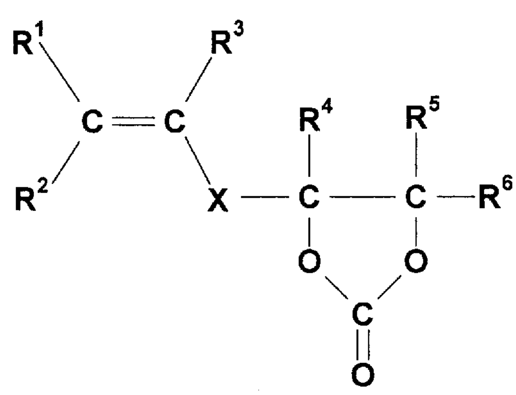

(3)

[formula 4]

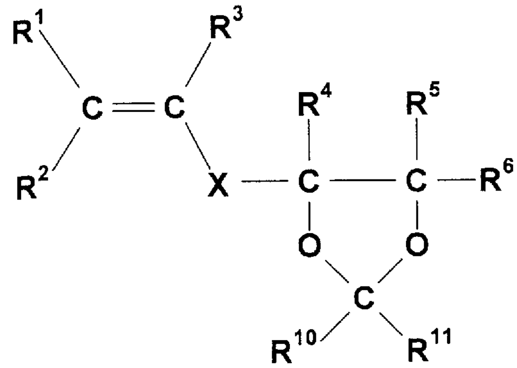

(4)

In the formulae 2, 3, and 4, each of $R^1$ to $R^6$is the same as one in the formula 1. $R^7$ and $R^8$ is individually hydrogen atom or $R^9$-CO-, wherein $R^9$ is alkyl group. Each of $R^{10}$ and $R^{11}$ is hydrogen atom or organic group.

A method disclosed in, for instance, Japanese Unexamined Patent Publication No. 2006-95825 may be employed for methods of (i), (ii), and (iii).

In particular, the method (i) is preferred in view of copolymerization reactivity and excellence in industrial handling. In addition, a compound where all of $R^1$ to $R^6$ are hydrogen atoms, X is single bond, and $R^7$ and $R^8$ are $R^9$-CO-, wherein $R^9$ is alkyl group, namely 3,4-diacyloxy-1-butene is preferable, and in particular, a compound wherein $R^9$ is methyl group, namely 3,4-diacetoxy-1-butene is more preferable.

The same manner saponification as one for producing PVA without 1,2-diol in a side chain can be employed for producing 1,2-diol containing PVA in a side chain.

When a spherical PVA particle is sterilized, it is sterilized with y-ray, pressure steam sterilization, immersion in hibitane solution (chlorhexidine gluconate solution), washing with sterile saline, and so on.

A spherical PVA particle having the desired average particle diameter is obtained optionally by physical sieving spherical PVA particles obtained by the above-mentioned method with standard mesh. The physical sieving is usable for controlling a particle diameter of the spherical PVA particle to be obtained. In order to obtain particles as small as a desired average particle diameter, it may use techniques for controlling particle diameter in production of spherical polyvinyl alcohol as disclosed in Japanese Unexamined Patent Publication No.S56-120707. The technique includes increasing stirring speed during saponification, setting viscosity of medium (c) such as liquid paraffin higher than one of polyvinyl ester solution (b), and controlling a ratio of medium (c) and polyvinyl ester solution (b).

[preparation of temporary embolic agent]

A temporary embolic agent used in the invention is in a form of dispersion, solution or paste of temporary embolic material, preferably, a dispersion or solution or paste in which the spherical PVA particle as described above is dispersed or dissolved in a medium.

The medium includes aqueous medium such as alcohol, purified water, saline, etc.; and non-aqueous medium such as iodinated fatty acid ester derived from opium oil. The PVA dispersion obtained after saponification in the above spherical PVA particle production may be used as it is. A dispersion obtained immediately after mixing spherical PVA particles with an aqueous medium may also be used because the dispersion in which partial or whole of spherical PVA particles are dispersed despite of using an aqueous medium.

A contrast agent may be also used for a medium. A contrast agent, which is in a form of liquid, is used for the purpose of confirmation whether blood flow is stopped or not. In addition, administration of a temporary embolic agent is usually examined using X-ray with a contrast agent. Accordingly, the contrast agent can also be used as the medium.

Examples of the contrast agent are contrast agent for urinary or blood vessel, contrast agent for MRI, contrast agent for alimentary canal and so on. Ordinarily, contrast agent for blood vessel, which is ionic water-soluble contrast agent or non-ionic water-soluble contrast agent, is used. Specific examples of the contrast agent are Iopamiron™ (Schering AG), Oiparomin™ (Fuji Pharma Co., Ltd.), Hexabrix™ (Terumo Corporation), Omnipaque™ (Daiichi Sankyo Co., Ltd), Visipaque™ (Daiichi Sankyo Co., Ltd.), Iomeron™ (Eisai Co., Ltd.), and Proscope™ (Mitsubishi Tanabe Pharma Corporation), which are normally aqueous solution. Mixing a contrast agent with PVA particles is preferably conducted immediately before administration. A temporary embolic agent thus prepared is in a form of dispersion of

spherical PVA particles, and has excellent catheter passing property.

In addition, an anticoagulant such as heparin, warfarin, and anti-platelet may be contained in the temporary embolic agent.

When such a temporary embolic agent is administered, spherical PVA particles in the temporary embolic agent start to be dissolved in the blood vessel and to become in a state of paste, thereby embolizing the blood vessel, that is, achievement of vascular blockage. The continuation of embolized state in the blood vessel for 1 hour or more causes necrosis of myocardial cells having supplied with nutrition through the embolized blood vessel. Subsequently, the PVA particles are dissolved in blood and/or body fluid and are excreted out of the body, thereby decreasing the viscosity of the paste, as a result, the state of vascular blockage is diminished in due course.

In the case that spherical PVA particles are used as a temporary embolic agent, whole of the spherical PVA particles are excreted out within a week or so, depending on the dissolving speed of the spherical PVA particles in the medium used for the embolic agent. Accordingly, the embolized blood vessel is revascularized to restart blood flow therein after certain period of time, and the blood reflow allows a catheter to be inserted in the blood vessel.
No generation of thrombus caused by blocking blood flow with spherical PVA particles can be confirmed with use of a contrast agent.

<Non-human animal model of myocardial infarction>

A non-human animal model of myocardial infarction of the present invention is a model created according to the inventive creation method.
In the model of myocardial infarction created by the method described above, cardiac muscles to which the blocked coronary artery has supplied nutrition are necrotized, and there is a pathological condition of so-called myocardial infarction.

However, in the coronary artery which induced myocardial infarction, an embolic agent used for blocking the vessel can be gradually dissolved in blood and disappeared after certain period of time, and moreover, the interruption of blood flow is not caused by a mechanically blocking device such as an ameroid ring, therefore, a catheter can be inserted again into the blood vessel at the site of myocardial infarction after the disappearance of an embolic agent.
Accordingly, such non-human animal models of myocardial infarction of the present invention may be used as the models for local therapy, drug screening for local treatment and studies on regenerative medicine.

The actual causes of myocardial infarction include obstruction or stenosis of an artery by coronary atherosclerosis and also clot formation which rapidly blocks a coronary artery, and in the case of the myocardial infarction due to the formation of a blood clot, subsequent thrombolysis and so on may restart the blood flow. Accordingly, non-human animal models of myocardial infarction of the present invention can also be used for studies on pathology and aspects of treatment, and screening for therapeutic agents for such myocardial infarction.

As non-human animals used in the model, mammals such as rats, mice, rabbits, pigs can be used, among them, pigs are preferably used. Pigs resemble humans in physiological and anatomical characteristics, and physiology on digestion and absorption, in particular, in heart morphology, distribution of coronary artery and endothelial structure of artery.

## EXAMPLES

Hereinafter, the present invention is described specifically with reference to examples, but the invention is not limited to the description unless exceeding its gist.
Incidentally, "parts" in the examples are on the weight basis, unless otherwise indicated.

[Measurement/evaluation method]

(1) Degree of saponification

A degree of saponification of a spherical PVA particle was measured according to JIS K-6726. The measurement was carried out by analyzing an amount of alkaline exhausted for hydrolysis of vinyl acetate residue and ester derived from 3,4-diacetoxy-1-butene.

(2) Average particle diameter

An average code length was adopted as an average particle diameter of a spherical PVA particle. The average code length was determined by adding 10 parts of spherical PVA particles to 100 parts of isopropyl alcohol being poor solvent for the highly saponified PVA, conducting a measurement under stirring using Lasentec M100 (particles inline

monitoring system, manufactured by Lasentec), and calculating as follows.
A region having a range of 0.8 to 1000 μm was divided into 38 channels of codes, and the number of particles in each channel was calculated, followed by calculating according to the following equation.

$$\text{Average code length} = \Sigma(Yi \times Mi2) / \Sigma Yi$$

Yi: the count number of particles monitored by Lasentec M100
Mi: the code length of each channel

(3) Solubility in water

One gram of spherical PVA particles classified into the range of 100 to 212 μm was charged into a 100ml beaker containing 70g of water and 4g of a contrast agent (IOMERON 300, Eisai Co., Ltd.), and was stirred for two minutes at room temperature, thereafter, the beaker was immersed in water bath with keeping the temperature at 37˚C. The number of spherical PVA particles is counted with stirring by use of LASENTEC M100F (Lasentec, taking-in time: 24.75 seconds). A residue rate, which corresponds to a ratio of particle numbers of three hours after measurement to immediately after the measurement, was adopted for solubility evaluation index.

(4) Average polymerization degree

An average polymerization degree was measured according to JIS K6726.

(5) Degree of crystallization (DSC method)

A heat of melting AH(J/g) was measured with Differential scanning calorimeter DSC7 (manufactured by PerkinElmer Japan Co., Ltd.) at a temperature between 30 and 250˚C with heating up at a rate of 20˚C/min and a value obtained by first run was used for calculating degree of crystallization according to the following equation.
In the equation below, 156 (J/g) is a value of energy of melting when a completely saponified unmodified PVA is 100% crystallized.

$$\text{degree of crystallization (\%)} = (\Delta H/156)\times 100$$

(6) Degree of true sphere (spherical degree coefficient)

With respect to individual PVA spherical particles in a visual field unit of the image obtained by scanning electron microscope, a length (L) and square of the particle contour are measured based on the image, and a circumference (M) of a circle having the same square as one of the particle is determined, thereafter, an average value of spherical degree coefficients (M/L) of the particles was calculated. Closer to 1 the spherical degree coefficient is, closer to true sphere the shape of the particle is.

(7) Confirmation of myocardial infarction

Diagnostic imaging of myocardial infarction was conducted by contrast cardiac MRI scans.
7cc of gadolinium-containing contrast agent for MRI (Omniscan Intravenous Injection 32% Syringe 10 mL, Daiichi Sankyo) was injected intravenously at 3.0 cc/sec, and after 15 minutes, the images were taken by using MRI device (Sigma EXCIE XI version 11.0, General Electoric (US)) at TI=250 to 300 msec. According to the literature (Kim RJ, Fieno DS, Parrish TB, et al., Relationship of MRI delayed contrast enhancement to irreversible injury, infarct age, and contractile function. Circulation 100: 1992-2002. 1999), the site of delayed excretion of the contrast agent was diagnosed based on its image as the focus of myocardial infarction.
Moreover, the pathological diagnosis of myocardial infarction was conducted by 2,3,5-triphenyl-2H-tetrazolium chloride (TTC) staining and hematoxylin and eosin staining. Immediately after the above diagnostic imaging of the focus of myocardial infarction, the animal was sacrificed, and the heart was extirpated, and then the heart were sliced in the same direction as the image of MRI, followed by immersion in TTC solution for 15 minutes, and the site with a color grossly changed to white was diagnosed as the focus of myocardial infarction. Furthermore, serial sections were prepared from the site in which the color was changed to white, fixed in formalin, followed by hematoxylin and eosin staining to

confirm the site with cardiomyocyte death, fibrillization and inflammatory cell infiltration as the focus of myocardial infarction.

[Preparation of spherical PVA particles]

Charged were 900g of vinyl acetate, 1440g of methanol, and 9g of 3,4-diacetoxy-1-butene into reaction can equipped with a reflux condenser, a dropping funnel, and a stirrer, and azobisisobutyronitrile was added at a proportion of 0.3 mol% to the vinyl acetate, and allowed to initiate polymerization upon heating while stirring under nitrogen gas flow. When the polymerization rate was reached at 98%, the polymerization was terminated by addition of 70 ppm of m-methoxy phenol. Subsequently, unpolymerized vinyl acetate monomer was removed by blowing methanol steam into the reaction system to obtain a methanol solution of copolymer.

The obtained methanol solution of the copolymer was diluted to a resin content of 40%. 100 parts of the diluted solution was charged into a reaction can with a stirrer, followed by addition of a saponification catalyst, NaOH (2 % methanol solution that is converted to the weight of Na) at a proportion of 3.2 mmol to vinyl acetate unit in polyvinyl acetate, with keeping a temperature at 30˚C while stirring.

Subsequently, 100 parts of liquid paraffin were added and the resulting mixture was stirred at a stirring speed of 300 rpm, thereby obtaining a dispersion where the polyvinyl acetate having a spherical shape is dispersed in liquid paraffin. The resulting dispersion was allowed to react while keeping a temperature at 30˚C and to be terminated after the lapse of time of 60 minutes. _Spherical PVA particles were separated by carrying out solid/ liquid separation by a centrifugal separator. The obtained particles were rinsed by an extraction method using ethyl acetate solution at a temperature of 50˚C and dried at a temperature of 80˚C for 24 hours by a vacuum dryer.

Thus obtained spherical PVA particle (primary saponified particle) in an amount of 100 parts was dispersed again in 500 parts of methanol solution, and then 20 parts of 2 % NaOH/methanol solution (that is converted to the weight of Na) as a saponification catalyst were added thereto, followed by a secondary saponification over 2 hours at 50˚C. After the secondary saponification, the spherical PVA particles were separated again by a centrifugal separator, rinsed by an extraction method using an ethyl acetate solution at 50˚C, and then dried at 80˚C for 24 hours using vacuum dryer.

The resultant spherical PVA particles had a saponification degree of 99.7 mol% and an average polymerization degree of 500. The content of 1,2-diol structural unit was 0.49 mol% as measured with $^1$H-NMR (internal standard material: tetramethylsilane) and calculated. The cloud point temperature of the PVA resin was 100˚C or more.

The particles were subjected to heat treatment of 120˚C for 30 minutes, thereafter obtaining spherical PVA particles. Thus produced spherical PVA particles had a degree of crystallization (DSC method) of about 62%, and solubility in water of 31.4% in terms of residue rate.

[Preparation of temporary embolic agent]

0.25g of the resulting spherical PVA particles used as a temporary embolic material, 5cc of OYPALOMIN (contrast agent manufactured by Fuji Pharma Co., Ltd.), and 1 cc of heparin were mixed to prepare a temporary embolic agent.

[Method for creating a model of myocardial infarction]

(1) Models No. 1 - 5 (models of left circumflex artery-related myocardial infarction)

A pig was sedated by intramuscular injection of a mixture of ketamine (10 mg/kg) and xylazine (2 mg/kg), and a tracheal tube was inserted to maintain the anesthetic drug (5% Isoflurane) at a concentration of 1 to 3%. The pig was placed in the supine position with his limbs bounded, and a femoral artery is exposed, followed by insertion of 8 Fr sheath. After heparin (100 U/Kg) was intravenously injected, a guiding catheter (Brite Tip 6 Fr. 100 cm: Hockey Stick, Johnson and Johnson Cordis (U.S.)) was placed in the entrance of a branch blood vessel of the left circumflex artery.

Subsequently, a balloon catheter (Gateway PTA Dilatation catheter 9.0 mm x 2.0 to 3.0 mm at 6 atm, Boston Scientific) was placed in the entrance of a branch blood vessel of the circumflex artery through the guiding catheter and the balloon was expanded to stop the blood flow of the targeted branch blood vessel of the circumflex artery, and then 0.1 cc of the temporary embolic agent prepared in the above formulation was manually administered through the balloon catheter to the branch blood vessel of the circumflex branch, followed by an additional administration of 0.5 cc of saline. 0.1 cc solution of the embolic agent and 0.5 cc of saline were administered repeatedly at intervals of 5 minutes, and the administration were repeated until stop of the blood flow was confirmed by angiography. The angiographs in this operation are shown in Fig. 1 and Fig. 2.

Fig. 1 shows the beginning of the administration and the arrows denote a branch blood vessel of the circumflex

artery. In Fig. 2 showing the photograph immediately before the completion of administration, the branch blood vessel (arrows in the figure) looks lighter, which confirmed the interruption of the blood flow by the embolic agent.

After the completion of the step for blocking the branch blood vessel, the animal was recovered from anesthesia. To prevent infection, an antibiotic was administered for 3 days after the surgery. One week after the step for blocking the branch blood vessel, a guiding catheter was placed in the entrance of the targeted coronary artery, more specifically main trunk of the left circumflex artery, then the balloon of over-the-wire balloon catheter (Gateway PTA Dilatation catheter, 9.0 mm x 2.0 to 3.0 mm at 6 atm, Boston Scientific) was projected from the tip and extended to stop the blood flow of the main trunk. Under such a condition, from the tip of the balloon catheter, 0.1 cc of the temporary embolic agent prepared in the above formulation was manually administered, followed by an additional administration of 0.5 cc of saline. 0.1 cc solution of the embolic agent and 0.5 cc of saline were administered repeatedly at intervals of 5 minutes, and the administrations were repeated until stop of the blood flow was confirmed by angiography. The angiographs in this operation are shown in Fig. 3 and Fig. 4.

In Fig. 3, left arrows denote the main trunk of the circumflex branch, and Fig. 4 confirms the interruption of the blood flow in the main trunk of the circumflex branch (right arrows in the figure).

After one week of the step to induce myocardial infarction (2 weeks after the preconditioning), an image provided by MRI scans is shown in Fig.5 and the macroscopic photograph of the extirpated heart is in Fig. 6.

In Fig. 5, it was confirmed that the area of delayed excretion of the contrast agent was consistent with the transflux area (arrows) of the blood vessel blocked. And Fig. 6 showed that the site with delayed excretion of the MRI contrast agent (arrows) was consistent with the area of myocardial necrosis. Therefore, it was confirmed that a model of myocardial infarction occurred in the circumflex artery in a pig was created.

The models from No. 2 to 5 were confirmed in the same manner (the photographs not shown).

(2) Models No. 6 and 7 (anterior descending artery-related myocardial infarction model)

In a similar manner as the model No. 1 except that the target coronary artery was changed to the anterior descending artery, a step to interrupt the blood flow of the branch blood vessel and a step to induce myocardial infarction were performed.

Fig. 7 and Fig. 8 show the photographs provided by MRI scans one week after the step to induce myocardial infarction. Fig. 7 and Fig. 8 are long-axis and short-axis images provided individually by MRI scans, and contrast MRI scans revealed the image of delayed excretion of the contrast agent in the transflux area (the part indicated by arrows) of the anterior descending artery, which resulted in the confirmation of the pathological condition of myocardial infarction.

(3) Models No. 8 and 9

A guiding catheter was placed just before the entrance of the main trunk of the anterior descending branch, then the balloon of the balloon catheter was projected from the tip and extended to block the blood flow, and at the same time, the temporary embolic agent was administered until the anterior descending artery was completely blocked. Fig. 9 is the photograph showing delayed excretion of the contrast agent due to the complete blockage in the lumen of the anterior descending branch (the part indicated by arrows in Fig. 9). However, in about 30 minutes after blocking blood flow, every animal was dead due to ventricular arrhythmia, therefore, a focus of myocardial infarction failed to be created.

INDUSTRIAL APPLICABILITY

According to the method of the invention, a myocardial infarction model can be created only by insertion of a catheter and administration of an agent, in other words, vascular blockage can be achieved by a less invasive surgery without thoracotomy, therefore, a non-human animal model of myocardial infarction can be created with a high survival rate. Accordingly, the inventive method is usable for efficient creation of a model using a large sized animal such as a pig, which resembles human.

In addition, the myocardial infarction model created by the method of the invention, is usable in a model for developing topical therapy, screening pharmaceuticals for topical therapy, studies on regenerative medicine, since a blood vessel at a site of myocardial infarction is not permanently embolized, but revascularized after certain period of time, therefore a catheter can be inserted again.

## Claims

**1.** A method for creating a non-human animal model of myocardial infarction comprising:

administering a temporary embolic agent to a branch blood vessel of a heart coronary artery to block blood flow through the branch blood vessel (referred to as "vascular branch blockage"); and
administering a temporary embolic agent to a target coronary artery which is intended to induce myocardial infarction after a definite period of time after the vascular branch blockage to block blood flow through the target coronary artery.

**2.** A method for creating a non-human animal model of myocardial infarction according to claim 1, wherein the branch blood vessel is a branch blood vessel of the target coronary artery.

**3.** A method for creating a non-human animal model of myocardial infarction according to claim 1 or 2, wherein the administration of the temporary embolic agent in at least one selected from the group consisting of said step for the vascular branch blockage and said step for blocking blood flow in the target coronary artery is carried out by administering a temporary embolic agent to the blood vessel with expanding a balloon catheter at a upstream of the blood vessel.

**4.** A method for creating a non-human animal model of myocardial infarction according to any one of claims 1 to 3, wherein the temporary embolic agent is in a form of dispersion or solution or paste in which spherical PVA particles are dispersed or dissolved in a medium.

**5.** A non-human animal model of myocardial infarction created by the method of any one of claims 1 to 4.

**6.** A non-human animal model of myocardial infarction comprising a pathological site of myocardial infarction wherein at least one blood vessel of a coronary artery is suffered from ischemia to cause necrosis of a myocardial cell but the blood vessel is able to be revascularized.

**7.** A non-human animal model of myocardial infarction according to claim 5 or 6, wherein the animal is a pig.

**8.** A method for blocking blood flow of a non-human animal **characterized by** administering spherical PVA particles in an amount sufficient to embolize a blood vessel to be intended to block blood flow therethrough while the blood flow is inhibited by inserting a balloon catheter to an entrance of the blood vessel and expanding the balloon catheter at the entrance.

[Fig. 1]

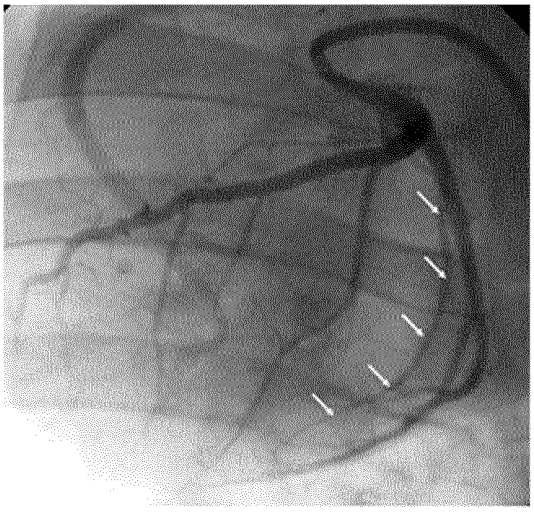

[Fig. 2]

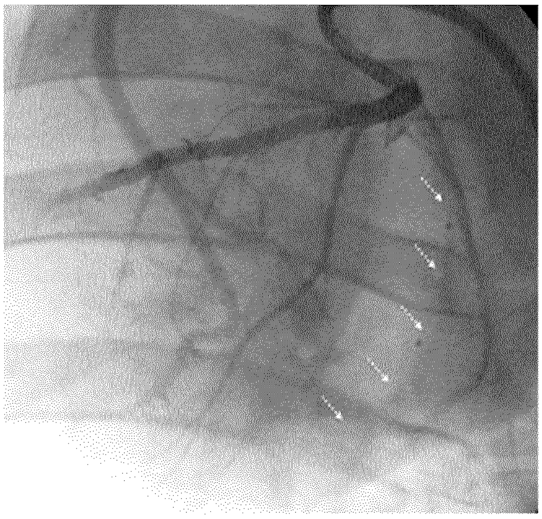

[Fig. 3]

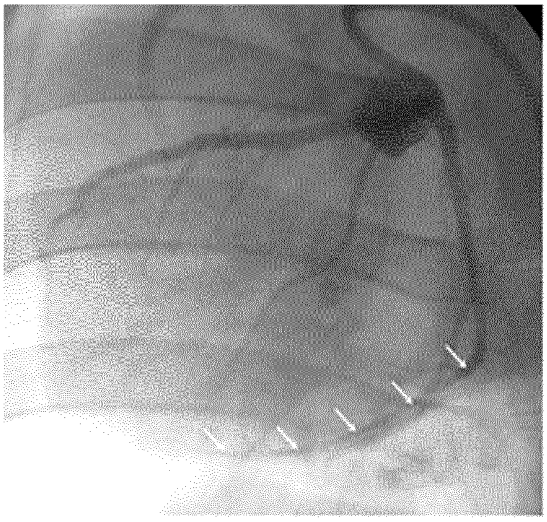

[Fig. 4]

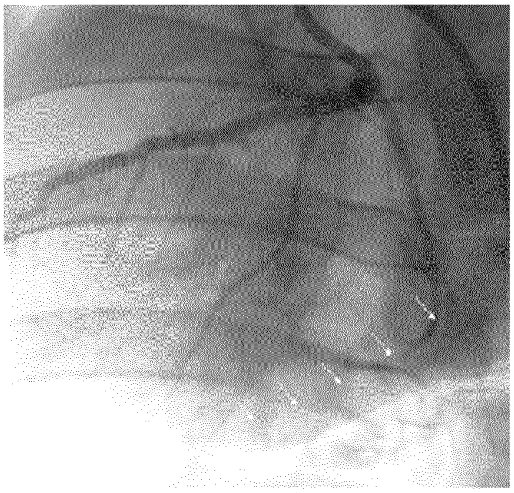

[Fig. 5]

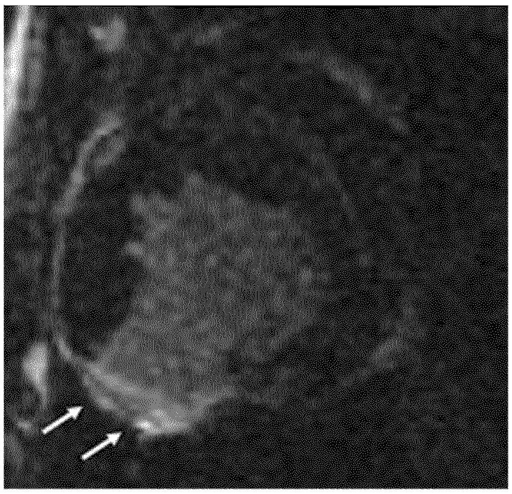

[Fig. 6]

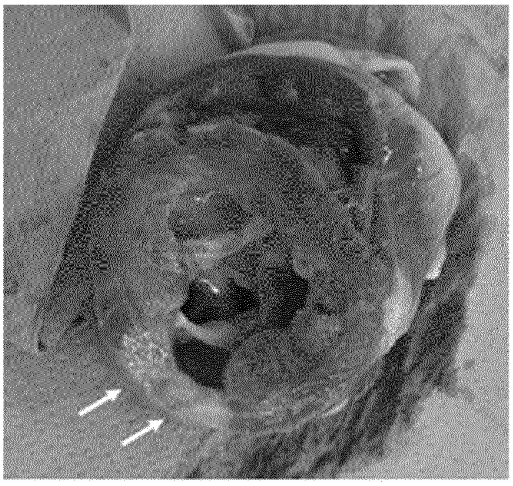

[Fig. 7]

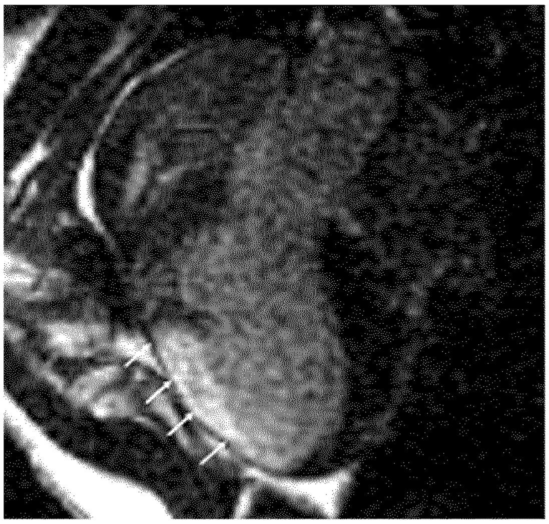

[Fig. 8]

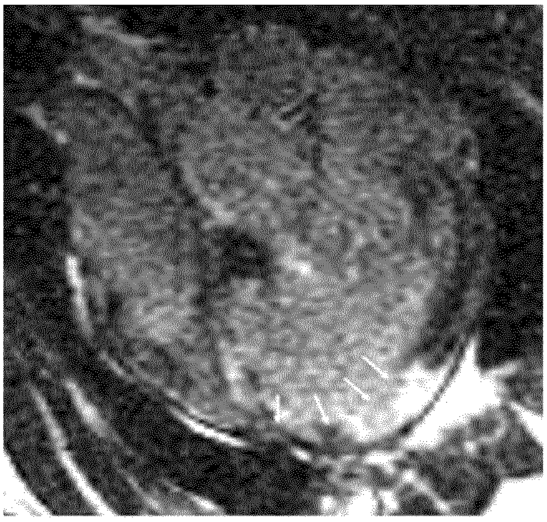

[Fig. 9]

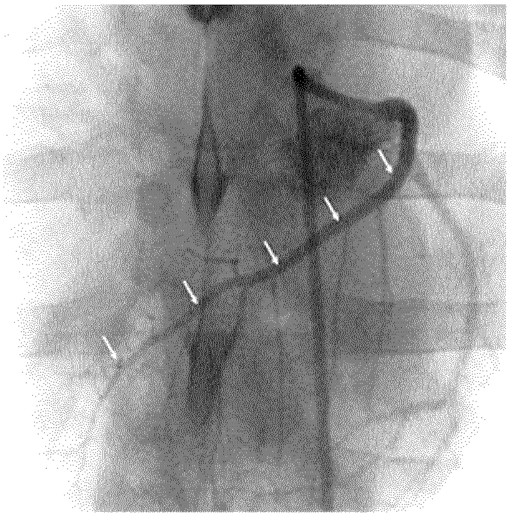

**INTERNATIONAL SEARCH REPORT**

International application No.
PCT/JP2010/060420

A. CLASSIFICATION OF SUBJECT MATTER
*A01K67/027*(2006.01)i

According to International Patent Classification (IPC) or to both national classification and IPC

B. FIELDS SEARCHED

Minimum documentation searched (classification system followed by classification symbols)
A01K67/027

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/BIOSIS/MEDLINE/WPIDS(STN), JSTPlus/JMEDPlus/JST7580(JDreamII), PubMed, CiNii

C. DOCUMENTS CONSIDERED TO BE RELEVANT

| Category* | Citation of document, with indication, where appropriate, of the relevant passages | Relevant to claim No. |
|---|---|---|
| Y | WO 2006/030737 A1 (Japan Health Sciences Foundation), 23 March 2006 (23.03.2006), claims; example 1 & US 2008/0295189 A1 & EP 1808071 A1 | 1-8 |
| Y | WO 2007/004484 A1 (The Nippon Synthetic Chemical Industry Co., Ltd.), 11 January 2007 (11.01.2007), examples & JP 2007-37989 A & EP 1900381 A1 | 1-8 |
| A | WO 1999/018861 A1 (SCIMED LIFE SYSTEMS, INC.), 22 April 1999 (22.04.1999), & JP 2001-519194 A & US 6074407 A & EP 1022988 A | 1-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 July, 2010 (02.07.10) | 13 July, 2010 (13.07.10) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2010/060420

C (Continuation). DOCUMENTS CONSIDERED TO BE RELEVANT

| Category* | Citation of document, with indication, where appropriate, of the relevant passages | Relevant to claim No. |
|---|---|---|
| A | WO 2001/089501 A1 (PROVASIS THERAPEUTICS), 29 November 2001 (29.11.2001), & US 2002/0141969 A1 & US 2002/0098150 A1 & EP 1283707 A & EP 1154723 A | 1-8 |
| A | Tetsuro SASANO et al., "Buta Shinkin Kosoku Model ni Taisuru cadioshere-derived cell o Mochiita Saibo Chiryo, Kei-Kandomyaku-teki Toyo no Shiteki Joken Kento to Saibo Chiryo ni yoru Shinkino Fuseimyaku Gensei no Hyoka", Dai 26 Kai Annual Meeting of the Japanese Society of Electrocardiology Program Shorokushu, 05 June 2009 (05.06.2009), pages S-3-323, 91 | 1-8 |
| P,X | Mitsuo SHIBUYA, Kaoru INOUE, "Amorphous Vinyl Alcohol-kei Jushi", Japan Plastics, 01 January 2010 (01.01.2010), vol.61, no.1, pages 56 to 62 | 1-8 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002291373 A
- JP 2005229927 A
- JP 2002209473 A
- WO 2006030737 A
- WO 983203 A
- JP 2004167229 A
- JP 2007037989 A
- JP S56120707 B
- JP 2006095825 A

### Non-patent literature cited in the description


- **Kim RJ ; Fieno DS ; Parrish TB et al.** Relationship of MRI delayed contrast enhancement to irreversible injury, infarct age, and contractile function. *Circulation,* 1999, vol. 100, 1992-2002